# FASCICULE DE BREVET EUROPEEN

(11) **EP 2 543 408 B1**
(45) Date de publication et mention de la délivrance du brevet: **11.10.2017**
(21) Numéro de dépôt: 12172957.8
(22) Date de dépôt: 21.06.2012
(51) Int. Cl.: A61M 16/06

(54) **Masque d'assistance respiratoire**
Maske zur Atmungsunterstützung
Assisted-breathing mask

(30) Priorité: 05.07.2011 FR 1102101
(43) Date de publication de la demande: 09.01.2013
(73) Titulaire: Boussignac, Georges, 92160 Antony (FR)
(72) Inventeur: Boussignac, Georges, 92160 Antony (FR)
(74) Mandataire: Jacobacci Coralis Harle

(56) Documents cités:
- WO-A1-2006/074513
- US-A- 3 330 273

## Description

La présente invention a pour objet un masque d'assistance respiratoire, nasal ou bucco-nasal, utilisable notamment sur des patients dont la respiration spontanée est absente ou insuffisante. EP1121953A1 divulgue les caractéristiques du préambule de la revendication 1. On connaît déjà des masques d'assistance respiratoire permettant d'amener, dans les poumons d'un patient, du gaz respiratoire provenant d'une source extérieure, lesdits masques respiratoires comportant :
- un corps creux, dont le volume interne définit une chambre pourvue d'une entrée de gaz respiratoire formée par un embout solidaire du fond du corps apte à être relié à ladite source, le corps creux étant destiné à être appliqué sur le visage du patient en enfermant le nez et/ou la bouche de celui-ci ;
- une ouverture qui constitue la sortie de gaz respiratoire et qui est destinée à être reliée à une voie respiratoire du patient ; et
- un organe d'appui souple - par exemple sous la forme d'un bourrelet gonflable ou d'un bourrelet en mousse à cellules fermées - qui borde le voisinage du contour de ladite ouverture et qui est destiné à prendre appui sur le visage du patient pour assurer l'étanchéité entre ladite sortie de gaz respiratoire et l'extérieur.

On sait en outre que la souplesse de l'organe d'appui confère au corps creux une certaine liberté d'orientation une fois le masque positionné sur le visage d'un patient. Autrement dit, l'organe d'appui assure une fonction d'orientation dans l'espace du corps creux, permettant une manipulation de ce dernier sans inconfort pour le patient.

Toutefois, malgré sa souplesse, un tel organe d'appui ne peut épouser parfaitement certaines irrégularités du visage du patient sur lequel il est appliqué, de sorte :
- qu'il se forme localement des fuites à l'origine de coûteuses pertes de gaz respiratoire ; et
- qu'il apparaît un risque que du gaz respiratoire, passant entre l'organe d'appui et le visage par suite des défauts d'étanchéité, pénètre dans les yeux du patient, engendrant des irritations oculaires et des conjonctivites.

Pour pallier ces défauts d'étanchéité, on augmente généralement la force d'application du masque sur le visage - par exemple à l'aide de sangles élastiques passant derrière la tête du patient - de manière à comprimer l'organe d'appui contre le visage.

Cependant, outre le fait qu'elle favorise l'apparition d'escarres, l'application vigoureuse du masque respiratoire sur le visage d'un patient provoque une compression importante de l'organe d'appui souple, ce qui tend à le rigidifier. En perdant de sa souplesse, l'organe d'appui n'assure plus qu'une orientation limitée du corps creux par rapport au visage.

Ainsi, plus on cherche à obtenir une étanchéité uniforme entre l'organe d'appui et le visage, plus on doit appliquer avec force le masque sur le visage, ce qui réduit d'autant l'amplitude du mouvement du corps creux par rapport au visage.

La présente invention a pour objet de remédier à ces inconvénients et, notamment, de permettre une certaine amplitude d'orientation du corps creux par rapport au visage d'un patient - lorsque le masque est en position - tout en garantissant une étanchéité uniforme entre le masque et le visage.

A cette fin, selon l'invention, le masque d'assistance respiratoire permettant d'amener, dans les poumons d'un patient, du gaz respiratoire provenant d'une source extérieure, ledit masque étant défini dans la revendication 1. Ainsi, grâce à l'invention, du gaz respiratoire peut traverser les perforations de la jupe pour tendre la membrane souple et la plaquer contre le visage du patient en s'adaptant à sa morphologie. Cela assure une étanchéité uniforme entre le visage et le masque, le long de l'ouverture de la chambre interne. Du fait qu'une étanchéité convenable est obtenue par application de la membrane, on peut réduire sensiblement la force d'application du masque respiratoire sur le visage du patient, supprimant ainsi le risque d'apparition d'escarres lors d'un port prolongé du masque tout en atténuant l'inconfort d'utilisation pour le patient. La force d'application du masque étant réduite, l'organe d'appui n'est pas, ou que très peu, comprimé, de sorte qu'il conserve toute sa souplesse et assure pleinement sa fonction d'orientation du corps creux par rapport au visage, une fois le masque en position. Ainsi, grâce à l'invention, la fonction d'orientation du corps creux est réalisée par l'organe d'appui, alors que l'étanchéité uniforme entre le masque et le visage est obtenue principalement par la membrane souple. Aucun compromis n'est désormais nécessaire entre l'orientation du corps creux et l'étanchéité du masque sur le visage.

De façon avantageuse, pour parfaire l'étanchéité réalisée par la membrane souple en assurant une distribution uniforme du gaz respiratoire, lesdites perforations sont régulièrement réparties le long de la circonférence de ladite jupe. Bien entendu, en variante, on pourrait envisager toute autre répartition désirée des perforations.

De préférence, lesdites perforations sont ménagées dans ladite jupe en regard de ladite membrane, pour garantir une application parfaite de la membrane contre le visage et pour éviter la formation de fuites.

En outre, chacune desdites perforations peut se présenter sous la forme d'un trou oblique pratiqué dans ladite jupe, de manière à orienter le gaz respiratoire vers ladite membrane, tout en limitant les turbulences à l'entrée des trous.

Par ailleurs, l'extrémité libre de ladite membrane s'étend de préférence vers l'intérieur de ladite ouverture, ce qui permet d'adapter aisément le masque sur plusieurs types différents de visage.

Ledit organe d'appui se présente sous la forme d'un bourrelet gonflable ou d'un bourrelet en mousse à cellules fermées. Il va de soi qu'il pourrait tout aussi bien prendre la forme d'un soufflet, ou bien encore toute autre forme appropriée.

Avantageusement, ladite jupe, souple ou semi-rigide, est conformée à la forme du visage pour faciliter l'adaptation et le maintien du masque sur ce dernier.

En outre, ladite membrane souple peut être réalisée dans un film plastique de quelques micromètres d'épaisseur.

Les figures du dessin annexé feront bien comprendre comment l'invention peut être réalisée. Sur ces figures, des références identiques désignent des éléments semblables.
La figure 1 représente schématiquement, selon une coupe axiale, un exemple de réalisation d'un masque d'assistance respiratoire conforme à la présente invention.
La figure 2 est une vue schématique agrandie de la zone A de la figure 1.

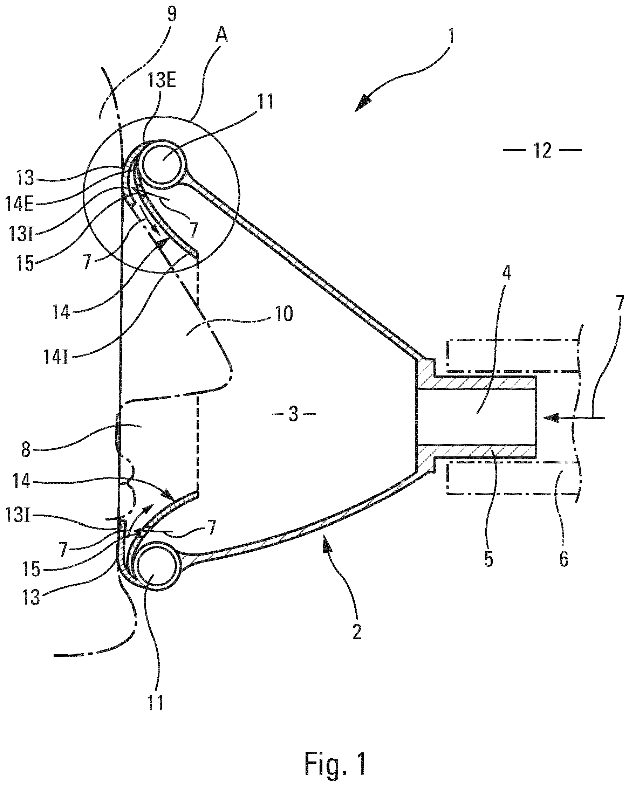

Le masque d'assistance respiratoire 1, conforme à la présente invention et représenté sur la figure, comporte une coque creuse 2 délimitant une chambre interne 3. Au fond de la coque 2 est aménagée une entrée 4 de gaz respiratoire, par exemple grâce à un embout tubulaire 5, solidaire de ladite coque 2, pouvant être reliée à une source de gaz respiratoire (non représentée), par exemple une bouteille sous pression, par une tubulure appropriée 6. Sur la figure 1, l'arrivée de gaz respiratoire frais est symbolisée par la flèche 7.

La chambre interne 3 comporte une sortie du gaz respiratoire formée par l'ouverture 8 de ladite coque 2. Cette dernière est destinée à être appliquée, par son ouverture 8, sur le visage 9 d'un patient (représenté en traits mixtes) en enfermant le nez 10 de celui-ci.

Le masque respiratoire 1 comporte en outre un organe d'appui sous la forme d'un bourrelet gonflable 11 à paroi mince, solidaire de la coque 2 et suivant le contour de l'ouverture 8 de cette dernière. Le bourrelet 11 est intercalé entre ladite ouverture 8 et le visage 9 du patient, lorsque la coque 2 est appliquée contre le visage 9.

Le masque respiratoire 1 comporte également une membrane souple 13, rapportée à une de ses extrémités 13E - par exemple par collage - sur le bourrelet gonflable 11 et apte à s'interposer entre ce dernier et le visage 9 du patient sur lequel il peut prendre appui. La membrane 13, réalisée dans un film plastique de quelques micromètres d'épaisseur, enveloppe partiellement le bourrelet 11 - notamment sa surface extérieure en regard du visage - et se prolonge, par son extrémité libre 131 vers l'intérieur de l'ouverture 8. La membrane 13 présente une souplesse plus importante que celle du bourrelet gonflable 11, ce qui lui permet de s'appliquer, avec ajustement, contre les aspérités du visage 9.

Le masque 1 comporte de plus une jupe interne multi-perforée 14 fixée par l'un de ses deux contours 14E - par exemple par collage - sur le bourrelet 11 et enveloppée par la membrane souple 13. La jupe 14 est de préférence en matériau semi-rigide - bien qu'elle pourrait, en variante, être souple - et conformée à la forme du visage 9 du patient. Elle est réalisée dans un matériau plastique, mais pourrait également être constituée d'une mousse à cellules fermées.

Les perforations 15 de la jupe 14 sont régulièrement réparties le long de la circonférence de celle-ci et sont agencées en regard de la membrane 13 pour permettre une distribution uniforme du gaz respiratoire et une application parfaite de la membrane 13 contre le visage 9.

Le contour libre 141 de la jupe 14 se prolonge à l'intérieur de la chambre interne 3 et participe à l'orientation d'une partie du flux d'air 7 vers les perforations 15 de la jupe 14.

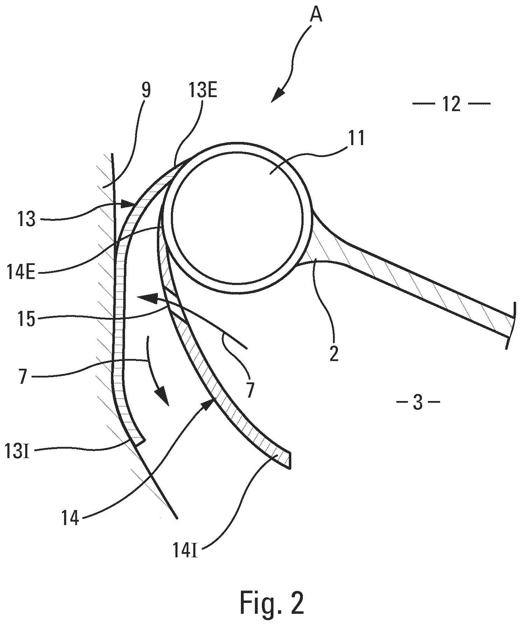

Comme le montrent les figures 1 et 2, les perforations 15 se présentent sous la forme de trous obliques ménagés dans la jupe 14, de manière à orienter le gaz respiratoire vers ladite membrane 13 tout en réduisant les turbulences au voisinage de l'entrée des trous.

Ainsi, lorsque le masque 1 est positionné sur le visage 9 du patient, la membrane souple 13 s'interpose entre le bourrelet 11 et le visage 9 pour établir une étanchéité uniforme sur le long de l'ouverture 8 et isoler la chambre interne 3 de l'extérieur 12.

En particulier, une partie du gaz respiratoire 7, qui pénètre dans la coque 2 par l'ouverture 4 (flèche 7) et passe dans la chambre interne 3, traverse les perforations 15 et vient tendre la membrane 13 afin de l'appliquer contre le visage 9. Ainsi, la membrane souple 13 s'adapte aisément à la morphologie du visage 9 du patient, par simple contact sur ce dernier, ce qui assure une étanchéité uniforme entre le visage 9 et le masque 1 le long de l'ouverture 8 sans nécessité une application vigoureuse du masque 1 sur le visage 9. Grâce à l'invention, l'étanchéité peut être obtenue par la seule membrane 13, bien qu'une participation du bourrelet 11 puisse également être envisageable.

De plus, puisque la membrane 13 est souple et élastique et qu'elle présente une extrémité libre 131 dans l'ouverture 8, le masque respiratoire 1 peut s'adapter automatiquement aux différentes morphologies des visages de dimensions variées, la membrane souple 13 tendue se conformant avec ajustement sur ces derniers.

## Revendications

1. Masque d'assistance respiratoire permettant d'amener, dans les poumons d'un patient, du gaz respiratoire (7) provenant d'une source extérieure, ledit masque (1) comportant :
- un corps creux (2), dont le volume interne définit une chambre interne (3) pourvue d'une entrée (4) de gaz respiratoire destinée à être reliée à ladite source ;
- une ouverture (8) qui constitue la sortie de gaz respiratoire et qui est destinée à être reliée à une voie respiratoire dudit patient ; et
- un organe d'appui souple (11) qui borde le contour de ladite ouverture (8) et qui est destiné à s'intercaler entre le visage (9) dudit patient et ledit corps creux (2), ledit organe d'appui souple étant un bourrelet gonflable ou un bourrelet en mousse à cellules fermées, le masque comportant en outre :
- une membrane souple (13) rapportée sur ledit bourrelet (11) et apte à s'interposer entre ledit bourrelet (11) et le visage (9) dudit patient ladite membrane souple (13) présentant une souplesse plus importante que celle du bourrelet (11) ; et, **caractérisé en ce que** le masque comporte
- une jupe interne multi-perforée (14) rapportée, par l'un de ses deux contours (14E), sur ledit bourrelet (11) et enveloppée, au moins partiellement, par ladite membrane souple (13), le contour libre (141) de ladite jupe se prolongeant en direction de ladite chambre interne (3),de sorte que du gaz respiratoire (7) est apte à traverser les perforations (15) de ladite jupe (14), depuis ladite entrée (4), pour tendre ladite membrane (13) et l'appliquer avec étanchéité contre le visage (9) du patient.

2. Masque selon la revendication 1, **caractérisé en ce que** lesdites perforations (15) sont régulièrement réparties le long de la circonférence de ladite jupe (14).

3. Masque selon l'une des revendications 1 ou 2, **caractérisé en ce que** lesdites perforations (15) sont ménagées dans ladite jupe (14) en regard de ladite membrane (13).

4. Masque selon la revendication 3, **caractérisé en ce que** chacune desdites perforations (15) se présente sous la forme d'un trou oblique pratiqué dans ladite jupe (14), de manière à orienter le gaz respiratoire vers ladite membrane (13).

5. Masque selon l'une des revendications 1 à 4, **caractérisé en ce que** l'extrémité libre (131) de ladite membrane s'étend vers l'intérieur de ladite ouverture (8).

6. Masque selon l'une des revendications 1 à 5, **caractérisé en ce que** ladite jupe (14) est conformée à la forme du visage (9).

7. Masque selon l'une des revendications 1 à 6, **caractérisé en ce que** ladite membrane souple (13) est réalisée dans un film plastique de quelques micromètres d'épaisseur.

## Patentansprüche

1. Maske zur Atemunterstützung, die es ermöglicht, ein von einer äußeren Quelle kommendes Atemgas (7) den Lungen eines Patienten zuzuführen, wobei die Maske (1)
- einen Hohlkörper (2), dessen inneres Volumen eine innere Kammer (3) definiert, die mit einem Einlaß (4) für Atemgas versehen ist, der dazu bestimmt ist, an die Quelle angeschlossen zu werden,
- eine Öffnung (8), die den Auslaß für Atemgas bildet und die dazu bestimmt ist, an einen Luftweg des Patienten angeschlossen zu werden, und
- ein nachgiebiges Andruckorgan (11), das den Rand der Öffnung (8) umrandet und das dazu bestimmt ist, sich zwischen das Gesicht (9) des Patienten und den Hohlkörper (2) zu setzen,
aufweist,
wobei das nachgiebige Andruckorgan eine aufblasbare Wulst oder eine Wulst aus Schaum mit geschlossenen Zellen ist, wobei die Maske außerdem
- eine an der Wulst (11) angebrachte und zwischen der Wulst (11) und dem Gesicht (9) des Patienten einfügbare flexible Membrane (13), die eine größere Flexibilität als jene der Wulst (11) hat, aufweist, und **dadurch gekennzeichnet, daß** die Maske
- eine innere, vielfach durchlöcherte Schürze (14) aufweist, die mit einem ihrer beiden Ränder (14E) auf der Wulst angebracht ist und wenigstens teilweise von der flexiblen Membrane (13) umhüllt ist, wobei sich der freie Rand (141) der Schürze in Richtung der inneren Kammer (3) fortsetzt, so daß das Atemgas (7) die Löcher (15) der Schürze (14) vom Einlaß (4) her durchdringen kann, um die Membrane (13) zu spannen und an das Gesicht (9) des Patienten dicht anzulegen.

2. Maske gemäß Anspruch 1, **dadurch gekennzeichnet, daß** die Löcher (15) regelmäßig über den Umfang der Schürze (14) verteilt sind.

3. Maske gemäß einem der Ansprüche 1 oder 2, **dadurch gekennzeichnet, daß** die Löcher (15) in der Schürze (14) gegenüber der Membrane (13) angeordnet sind.

4. Maske gemäß Anspruch 3, **dadurch gekennzeichnet, daß** jedes der Löcher (15) in Form eines in der Schürze (14) gebildeten schrägen Lochs vorliegt, um das Atemgas zur Membrane (13) hin zu lenken.

5. Maske gemäß einem der Ansprüche 1 bis 4, **dadurch gekennzeichnet, daß** sich das freie Ende (131) der Membrane zum Inneren der Öffnung (8) hin erstreckt.

6. Maske gemäß einem der Ansprüche 1 bis 5, **dadurch gekennzeichnet, daß** die Schürze (14) an die Form des Gesichts (9) angepaßt ist.

7. Maske gemäß einem der Ansprüche 1 bis 6, **dadurch gekennzeichnet, daß** die flexible Membrane (13) aus einem Plastikfilm mit einer Dicke von einigen Mikrometern hergestellt ist.

## Claims

1. A respiratory assistance mask allowing to bring, in the lungs of a patient, respiratory gas (7) from an external source, said mask (1) comprising:
- a hollow body (2), the internal volume of which defines an internal chamber (3) provided with a respiratory gas inlet (4) adapted to be connected to said source;
- an opening (8) forming the respiratory gas outlet and which is adapted to be connected to a respiratory tract of said patient; and
- a flexible bearing member (11) bordering the contour of said opening (8) and which is adapted to be interposed between the face (9) of said patient and said hollow body (2), said flexible bearing member being an inflatable bead or a closed pore foam bead,
the mask further comprising:
- a flexible membrane (13) inserted on said bead (11) and being able to be interposed between said bead (11) and the face (9) of said patient, said flexible membrane (13) having a bigger flexibility that the one of the bead (11); and,
**characterized in that** the mask comprises a multi-perforated internal skirt (14) inserted, thru one of the two contours (14E) thereof, on said bead (11) and surrounded, at least partially, by said flexible membrane (13), the free contour (141) of said skirt extending in the direction of said internal chamber (3), so that respiratory gas (7) is adapted to cross the perforations (15) of said skirt (14), from said inlet (4), so as to stretch said membrane (13) and apply it tightly against patient's face (9).

2. The mask according to claim 1, **characterized in that** said perforations (15) are regularly distributed along the circumference of said skirt (14).

3. The mask according to one of claims 1 or 2, **characterized in that** said perforations (15) are arranged in said skirt (14) opposite said membrane (13).

4. The mask according to claim 3, **characterized in that** each of said perforations (15) is present under the shape of an oblique hole arranged in said skirt (14), so as to orient the respiratory gas toward said membrane (13).

5. The mask according to one of claims 1 to 4, **characterized in that** the free end (131) of said membrane extends toward the inside of said opening (8).

6. The mask according to one of claims 1 to 5, **characterized in that** said skirt (14) is shaped to the form of the face (9).

7. The mask according to one of claims 1 to 6, **characterized in that** said flexible membrane (13) is made in a plastic film of a thickness of a few microns.
